# EUROPEAN PATENT APPLICATION

(11) **EP 3 534 281 A1**
(43) Date of publication of application: **04.09.2019**
(21) Application number: 17865774.8
(22) Date of filing: 31.10.2017
(51) Int. Cl.: G06F 19/24, C12M 1/34, C12Q 1/68

(54) **DISEASE DEVELOPMENT DETERMINATION DEVICE, DISEASE DEVELOPMENT DETERMINATION METHOD, AND DISEASE DEVELOPMENT DETERMINATION PROGRAM**

(30) Priority: 31.10.2016 JP 2016213690
(71) Applicant: Preferred Networks, Inc., Tokyo 100-0004 (JP)
(72) Inventor: OKANOHARA, Daisuke, Tokyo 100-0004 (JP); OONO, Kenta, Tokyo 100-0004 (JP); OTA, Nobuyuki, San Mateo California 94402 (US); HAMZAOUI, Karim, Tokyo 100-0004 (JP); AKIBA, Takuya, Tokyo 100-0004 (JP)
(74) Representative: Noble, Nicholas
(86) International application number: PCT/JP2017/039363
(87) International publication number: WO 2018/079840

(57) **Abstract**

To enable disease affection determination by using a neural network to perform learning using data of the expression levels of biomarkers, and to enable extraction of a feature biomarker for a disease by the neural network.

Sample data in which respective expression levels of a plurality of types of biomarkers are recorded for each individual is acquired, a learned model in which affection of diseases is determinable obtained in advance by performing machine learning using training data is generated, a plurality of sample data to which label information of disease affection is attached is input to the learned model and calculation is performed, the degrees of importance of respective feature of a plurality of biomarkers obtained with the learned model are quantified by affection determination calculation, for each sample data, and a predetermined number of biomarkers are extracted as feature biomarkers regarding the disease on the basis of the quantified degrees of importance of all the sample data for each biomarker.

## Description

### Technical Field

The present invention relates to a technique for performing disease affection determination by using a neural network to perform learning using data of expression levels of miRNAs, and extracting a miRNA that serves as a feature biomarker for a disease by the neural network.

### Background Art

Conventionally, techniques have been proposed for diagnosing diseases focusing on expression levels of microRNAs (miRNAs) in a sample derived from an organism . A miRNA is a functional nucleic acid composed of a single-stranded RNA molecule with a length of 21-25 bases and has a function to suppress translation of various genes having a target site complementary to itself, and is known to control basic biological functions such as generation, differentiation, and proliferation of a cell, cell death, and the like. 2500 or more types of human miRNAs have been currently discovered. Researches is being conducted on diagnosis and early detection of specific diseases, focusing on the fact that the expression level of a miRNA, among the vast variety of miRNAs, varies between an individual affected with the specific disease and an unaffected individual.

Patent Literature 1 is an example of a diagnostic tool for diagnosing a specific disease using a miRNA. Patent Literature 1 proposes a method for using a specific miRNA as a biomarker of hypopharyngeal cancer, a method for determining hypopharyngeal cancer, a determination kit for hypopharyngeal cancer, and the like.

### Citation List

### Patent Literature

Patent Literature 1: JP 2011-72229 A

### Summary

### Technical Problem

In Patent Literature 1, the miRNA from a hypopharyngeal cancer tissue and the miRNA from a hypopharyngeal normal tissue are compared, abnormal expression of a specific miRNA is found in the hypopharyngeal cancer tissue, and the specific miRNA is used as a biomarker for diagnosis of hypopharyngeal cancer. The conventional diagnosis using miRNAs finds and uses a miRNA related to a certain disease, and even in actual diagnosis, diagnosis is performed on the basis of the expression level of the miRNA related to the disease.

Although the method for performing diagnosis focusing on only the miRNA related to a disease can perform diagnosis with a certain degree of accuracy, the problem is that a positive case for the disease can exist even through a significant difference that can be diagnosed as being positive does not appear in the value of the miRNA of interest. Such problem may exist because it is necessary to set a threshold value about the value of miRNA of interest and to conduct diagnosis but it can be said that this is a problem occurring when diagnosis is performed focusing on only a few number of miRNAs. However, there is a problem that using all of the data of enormous miRNAs for diagnosis by the same technique is not easy.

In Patent Literature 1, the miRNA from a hypopharyngeal cancer tissue and the miRNA from a hypopharyngeal normal tissue are compared and the specific miRNA is extracted, and such a method for finding a feature miRNA by the method for comparing the actual diseased tissues is effective. However, improvement of diagnosis accuracy by effectively using all the data of the expression levels of 2500 or more types of miRNAs is not possible by the method for determining, by a human, whether a difference is significant when comparing the expression levels of individual miRNAs.

The present invention has been made in view of the above problem, and an object of the present invention is to provide a disease affection determination technique that enables disease affection determination by causing a neural network to perform learning using data of expression levels of biomarkers such as miRNAs, and to provide an extraction technique for a feature of a disease that enables extraction of a feature biomarker for a disease by the neural network.

### Solution to Problem

A disease affection determination device according to the present invention includes a sample data acquisition unit configured to acquire sample data including respective expression levels of biomarkers including a plurality of types of miRNAs in a human-derived sample, a learned model in which affection of diseases is determinable obtained in advance by performing machine learning using training data, and an affection determination unit configured to perform affection determination for the sample data on the basis of the degree of importance of each biomarker, using the learned model.

A disease affection determination device according to the present invention includes a sample data acquisition unit configured to acquire sample data including respective expression levels of biomarkers including a plurality of types of miRNAs in a human-derived sample, a learned model in which affection of diseases is determinable obtained in advance by performing machine learning using training data, an importance calculation unit configured to input the sample data to the learned model to quantify the degree of importance of each biomarker, and an affection determination unit configured to perform affection determination for the sample data from the degree of importance.

Further, the disease affection determination device according to the present invention includes a feature extraction unit configured to extract a feature biomarker regarding the disease on the basis of the degree of importance, wherein the affection determination is performed on the basis of feature importance that is the degree of importance of each feature biomarker in a case of performing disease determination only with the extracted feature biomarker.

Further, the disease affection determination device according to the present invention includes a feature extraction unit configured to extract a feature biomarker regarding the disease on the basis of the degree of importance, and a feature importance calculation unit configured to quantify feature importance that is the degree of importance of each feature biomarker in a case of performing disease determination only with the extracted feature biomarker, wherein the affection determination unit performs the affection determination from the feature importance.

Further, in the disease affection determination device according to the present invention, the importance calculation unit quantifies the degrees of importance of feature of respective biomarkers by a process of calculating a loss function Lᵢ regarding the i-th sample data, using the learned model, for each sample data, a process of performing error back propagation with a value Lᵢ of the loss function as a starting point and calculating a gradient gᵢⱼ = ∂Lᵢ/∂xⱼ regarding a feature xⱼ corresponding to each of a plurality of types of biomarkers of the sample i, and a process of obtaining an absolute value of a sum of gradients about all the samples as the degree of importance Sⱼ = |∑_{i}gᵢⱼ| of the feature.

Further, in the disease affection determination device according to the present invention, the training data is the sample data to which label information as to whether individuals are affected with diseases is attached.

Further, in the disease affection determination device according to the present invention, generation of the learned model is performed after a whitening process is performed, the whitening process being of linear transformation of each dimension such that an average over the entire training data becomes 0 and the variance becomes 1, for each dimension of a feature vector of the training data.

A disease affection determination method according to the present invention includes the steps of acquiring sample data including respective expression levels of biomarkers including a plurality of types of miRNAs in a human-derived sample, generating a learned model in which affection of diseases is determinable obtained in advance by performing machine learning using training data, and performing affection determination for the sample data on the basis of the degree of importance of each biomarker, using the learned model.

A disease feature extraction device according to the present invention includes a sample data acquisition unit configured to acquire sample data in which respective expression levels of biomarkers including a plurality of types of miRNAs in a human-derived sample are recorded for each individual, an affection determination unit including a learned model in which affection of diseases is determinable obtained in advance by performing machine learning using training data, and a feature extraction unit configured to input a plurality of sample data to which label information of disease affection is attached, to the affection determination unit to determine affection, to quantify the degrees of importance of respective feature of a plurality of biomarkers obtained with the learned model by affection determination calculation, for each sample data, and to extract a predetermined number of biomarkers as feature biomarkers regarding the disease on the basis of numerical values of the degree of importance of the plurality of sample data, for each biomarker.

A disease feature extraction method according to the present invention includes the steps of acquiring sample data in which respective expression levels of biomarkers including a plurality of types of miRNAs in a human-derived sample are recorded for each individual, generating a learned model in which affection of diseases is determinable obtained in advance by performing machine learning using training data, and inputting a plurality of sample data to which label information of disease affection is attached, to the learned model to determine affection, quantifying the degrees of importance of respective feature of a plurality of biomarkers obtained with the learned model by affection determination calculation, for each sample data, and extracting a predetermined number of biomarkers as feature biomarkers regarding the disease on the basis of numerical values of the degree of importance of the plurality of sample data, for each biomarker.

### Advantageous Effects of Invention

According to the present invention, a learned model is generated by performing machine learning while updating parameters in the process of learning by a neural network. Therefore, even if a human does not recognize existence of a miRNA related to a disease in advance, affection determination can be performed with high accuracy.

Further, according to the present invention, determination of malignant tumor and benign tumor, which has been difficult by conventional test methods, can be performed with high accuracy.

Further, according to the present invention, a plurality of sample data to which label information of affected individuals is attached is input to the generated learned model and affection determination is calculated, the degree of importance of the sample data is obtained in the process of calculation, an absolute value of a sum of the degrees of importance of all the sample data is obtained, feature of the sample data are ranked on the basis of the absolute value of the sum of the degrees of importance, and biomarkers corresponding to a predetermined number of feature from the top are extracted as feature biomarkers regarding the disease. Therefore, important miRNAs in the disease affection determination can be extracted as feature miRNAs. The processing capacity required for a computer can be decreased and the processing speed can be improved while accuracy of affection determination is improved by use of the extracted feature biomarkers.

### Brief Description of Drawings

Fig. 1 is a block diagram illustrating a configuration of a disease affection determination device 10 according to the present invention.
Fig. 2 is an explanatory diagram illustrating a concept of learning in a neural network.
Fig. 3 is a flowchart illustrating a flow of a learning process in the disease affection determination device 10.
Fig. 4 is a flowchart illustrating a flow of a feature extraction process in the disease affection determination device 10.
Fig. 5 is a table illustrating affection determination accuracy of when the present invention is applied for various diseases.
Fig. 6 is a block diagram illustrating a configuration of a disease affection determination device 22 that employs a stacking technique.

### Description of Embodiments

### [First Embodiment]

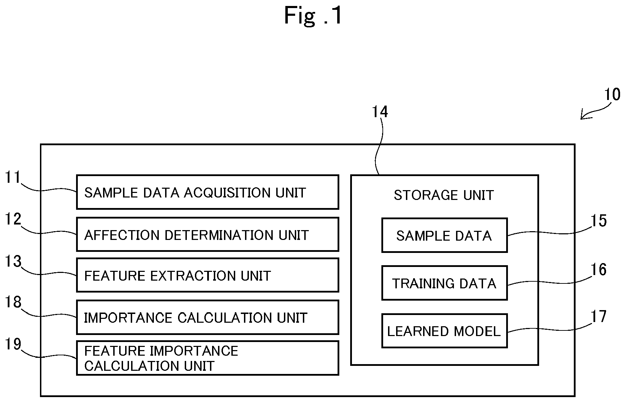

Hereinafter, an example of a disease affection determination device according to the first embodiment will be described with reference to the drawings. Fig. 1 is a block diagram illustrating a configuration of a disease affection determination device 10 according to the present invention. Note that the disease affection determination device 10 may be a device designed as a dedicated machine and affection may be realized by a general computer. In this case, the disease affection determination device 10 is furnished with a central processing unit (CPU), a graphics processing unit (GPU), a memory, and a storage such as a hard disk drive (not illustrated), which are supposed to be generally included in a general computer. It goes without saying that various processes are executed by a program in order to cause these general computers to function as the disease affection determination device 10 of the present example.

The disease affection determination device 10 includes at least a sample data acquisition unit 11, an affection determination unit 12, a feature extraction unit 13, and a storage unit 14.

The sample data acquisition unit 11 has a function to acquire sample data in which expression levels of respective biomarkers including a plurality of types of miRNAs in a human-derived sample are recorded for each individual. A human-derived sample refers to a sample derived from a human being, which may include biomarkers such as miRNAs of blood, a body fluid, a cell culture medium, and the like. Any technique for detecting the biomarkers such as the miRNAs from these samples may be used, but a technique capable of detecting all the detectable biomarkers such as miRNAs as much as possible is more preferred. A detection device for the biomarkers may be built in the disease affection determination device 10 or the sample data detected at an outside may be acquired by the sample data acquisition unit 11 through a communication network. The sample data for each individual has, for example, data items for 2500 or types more of miRNAs, and each item of the miRNAs is configured from numerical data representing an expression level per unit volume.

The affection determination unit 12 includes a learned model in which affection of diseases is determinable obtained in advance by performing machine learning using training data, and has a function to determine whether the individual sample data is affected with a disease, using the learned model. The training data refers to sample data to which label information as to whether affected with diseases is attached. To generate the learned model, it is favorable to have a plurality of sample data of affected individuals and a plurality of sample data of unaffected individuals. Note that, in the following description, description will be given using a case in which the machine learning is learning by a neural network as an example, but the embodiment is not limited to the case and various types of machine learning are applicable.

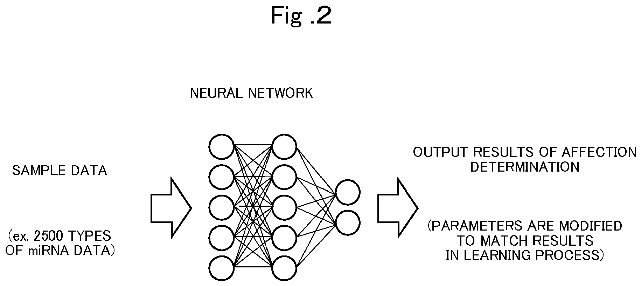

Fig. 2 is an explanatory diagram illustrating a concept of learning in a neural network. As illustrated in Fig. 2, in the learning by the neural network, the neural network is configured to be able to obtain the training data (sample data with label information) as an input and an affection determination result as an output. As actual learning by the neural network, for example, causing the neural network to perform a process of obtaining a loss function, and learning to perform disease affection determination from a value of the loss function can be considered. Parameters of the neural network are corrected from a difference between input data and the determination result, learning is performed to improve the determination accuracy, and the learned model is obtained. Examples of the neural net referred to here include Feedforward, CNN, VAE, GAN, and AAE.

An importance calculation unit 18 has a function to calculate the degree of importance that serves as a guide for how much a value of each biomarker in the sample data influences the affection determination when performing the affection determination for the sample data, using the learned model in the affection determination unit 12. Calculation of the degree of importance is the same as quantification of the degree of importance in the feature extraction unit 13 described below. Note that, in a case where the affection determination of the sample data is performed in the affection determination unit 12, it is also possible to input the sample data to the learned model and output only the affection determination result of the disease. Even in that case, the degree of importance is calculated and determination is made in the learned model, but there may be a case where the importance calculation unit 18 does not function independently. That is, in the present invention, the case where the affection determination is performed in the affection determination unit 12 includes a case where the importance calculation unit 18 functions as an internal process of the affection determination unit 12.

The feature extraction unit 13 has a function to extract feature biomarkers regarding diseases. The feature biomarker is a biomarker effective for determining an affected individual and an unaffected individual with the disease. A method for extracting the feature biomarkers is inputting a plurality of sample data to which label information of affected diseases is attached to the learned model learned in the affection determination unit 12 and performing affection determination, quantifying the degrees of importance of respective feature of a plurality of biomarkers obtained in the learned model by calculation of affection determination for each sample data, obtaining a sum of the quantified feature of the plurality of sample data for each biomarker, and extracting a predetermined number of biomarkers from ones having a large sum value as the feature biomarkers regarding the disease.

To be more specific, in the feature extraction unit 13, the degrees of importance of feature of respective biomarkers are quantified by a process of calculating a loss function Lᵢ regarding the i-th sample data, using the learned model, for each sample data, a process of performing error back propagation with a value Li of the loss function as a starting point and calculating a gradient gᵢⱼ = ∂Lᵢ/∂xⱼ regarding a feature xⱼ corresponding to each of a plurality of types of biomarkers of the sample i, and a process of obtaining an absolute value of a sum of gradients about all the samples as the degree of importance Sⱼ = |∑_{i}gᵢⱼ| of the feature, the biomarkers are ranked in descending order of the degree of importance, and a predetermined number of biomarkers from the top, for example, 100 biomarkers are extracted as the feature biomarkers.

A feature importance calculation unit 19 has a function to calculate feature importance that serves as a guide for how much the value of each feature biomarker influences the affection determination when only an extracted biomarker is employed as an item of input data and the affection determination is performed, when the feature biomarker is extracted in the feature extraction unit 13. In a case where the biomarkers are ranked in descending order of the degree of importance and a predetermined number of biomarkers from the top, for example, 100 biomarkers are extracted as the feature biomarkers, a process of performing affection determination using the 100 biomarkers as inputs is learned by the neural network, the learned model in the case of the 100 feature biomarkers is generated, and in a case where the affection determination of the sample data is performed by the affection determination unit 12 using the learned model, the feature importance is calculated by the feature importance calculation unit 19, and the affection determination is performed. It is also possible to input the sample data to the learned model and output only the affection determination result of the disease, similarly to the case of the importance calculation unit 18 described above. Even in that case, the feature importance is calculated and determination is made in the learned model, but there may be instances the feature importance calculation unit 19 does not function independently. That is, in the present invention, the case where the affection determination is performed in the affection determination unit 12 includes a case where the feature importance calculation unit 19 functions as an internal process of the affection determination unit 12.

The storage unit 14 has a function to store data that is used in the disease affection determination device 10 and data obtained as a processing result. To be specific, as illustrated in Fig. 1, at least sample data 15 acquired in the sample data acquisition unit 11, training data 16 to which label information as to whether affected with diseases in the sample data is attached, a learned model 17 generated by machine learning using the training data, and the like are stored.

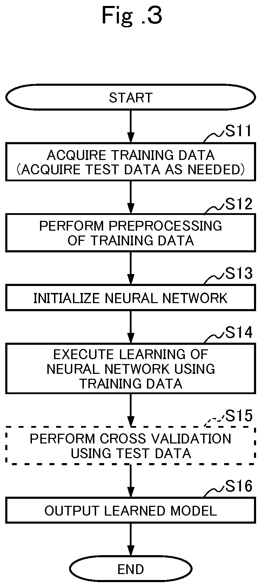

Next, a flow of processing in the disease affection determination device 10 according to the present invention will be described with reference to the drawings. Fig. 3 is a flowchart illustrating a flow of a learning process in the disease affection determination device 10. To perform the affection determination of diseases in the affection determination unit 12 of the disease affection determination device 10, the learned model needs to be generated by performing learning by the neural network in advance. Generation of the learned model may be performed by the affection determination unit 12 or a learned model that was separately generated may be used by the affection determination unit 12 after stored in the storage unit 14.

In Fig. 3, first, the generation of the learned model begins with acquiring the training data (step S11). In addition, test data is also acquired as necessary. The test data is sample data to which label information as to whether affected with diseases is attached, similar to the training data, and is sample data different from the training data. Preprocessing is performed on the acquired training data (step S12). In the preprocessing, a whitening process of linearly transformation of each dimension performed, such that an average over the entire training data becomes 0 and the variance becomes 1, for each dimension of a feature vector of the training data. Next, each parameter of the neural network is initialized (step S13). As a method of initialization, for example, a method of initializing each parameter by a random number is conceivable. After that, the training data is input to the initialized neural network and learning is performed(step S14). Learning is carried out to improve the determination accuracy by appropriately modifying the parameters such that the determination results of the affection determination matches the label information of the training data. After learning, to measure the determination accuracy, cross validation may be performed using the test data (step S15). The learning is terminated at the time when the learned model secured with the determination accuracy is obtained, the learned model is output and the process is terminated (step S16).

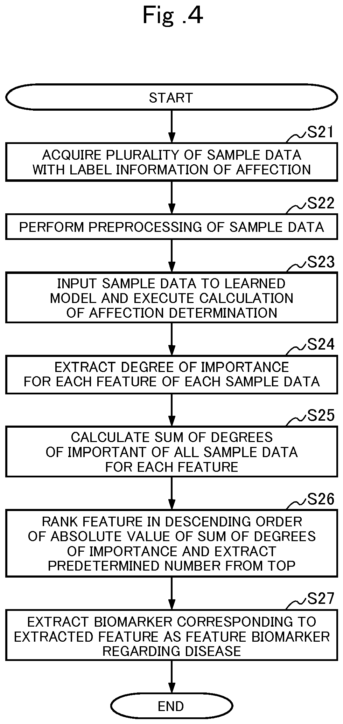

Fig. 4 is a flowchart illustrating a flow of a feature extraction process in the disease affection determination device 10. In Fig. 4, in disease feature extraction, first, a plurality of sample data to which label information indicating affected individuals is obtained (step S21). Preprocessing is performed for the plurality of acquired sample data (step S22). In the preprocessing, a whitening process of linearly transforming each dimension such that an average over the entire sample data becomes 0 and a variance becomes 1, for each dimension of a feature vector of the sample data, is performed. Next, the sample data is input to the learned model and calculation of the affection determination is executed (step S23). The calculation for the affection determination is, for example, calculation of a loss function. For each sample data, the degree of importance is extracted for each feature of the sample data (step S24). In the extraction of the degree of importance, a gradient relating to each feature of the sample data is calculated, and the magnitude of the gradient is quantified as the degree of importance, for example. Then, for each feature, a sum of the degrees of importance of all the sample data is calculated (step S25). The feature are ranked in descending order of absolute value of the sum of the degrees of importance, and a predetermined number of feature are extracted from the top (step S26). A biomarker corresponding to the extracted feature is extracted as the feature biomarker regarding the disease and the process is terminated (step S27).

As described above, according to the disease affection determination device 10 of the present invention, the learned model is generated by performing learning by the neural network, using the training data having data items of a plurality of types (2500 types or more, for example) of miRNAs, and the disease affection determination is performed using the learned model, and thus the learning is performed while the parameters are updated such that the expression levels of the miRNAs that are significant for the affection determination in the process of learning by the neural network influences the determination, whereby the affection determination can be accurately performed even if a human does not recognize existence of the miRNA related to the disease in advance.

Further, according to the disease affection determination device 10 of the present invention, a plurality of sample data to which label information of affected individuals is attached is input to the generated learned model and affection determination is calculated, the degree of importance of each feature of the sample data is obtained in the process of calculation, an absolute value of a sum of the degrees of importance of all the sample data is obtained for each feature, feature of the sample data are ranked on the basis of the absolute value of the sum of the degrees of importance, and biomarkers corresponding to a predetermined number of feature from the top are extracted as feature biomarkers regarding the disease, whereby important miRNAs in the disease affection determination can be extracted as feature miRNAs.

An advantage of extracting the feature biomarker is that the processing capacity required for a computer can be decreased and the processing speed can be improved while accuracy of the affection determination is maintained. Specifically, for example, the learned model that has performed learning on the basis of data of the expression levels of 2500 or more types of miRNAs enables highly accurate affection determination on the one hand, very high processing capacity is required for the computer for calculation processing and the calculation processing time is also long on the other hand. Therefore, for example, if top 100 feature miRNAs are extracted on the basis of the degree importance, learning is performed by the neural network with the sample data having the top 100 miRNAs as the data items to generate the learned model, and the affection determination is performed using the learned model, there is an advantage that the affection determination can be performed with accuracy comparable to the case of the affection determination based on 2500 types, the processing capacity of a computer for calculation processing can be decreased and the calculation processing time can be shortened.

As an example of accuracy improvement, in a conventional method of diagnosing breast cancer using five types of miRNAs, the diagnostic accuracy was 89%, whereas in the affection determination technique according to the present invention using 2500 types of miRNAs, diagnosis of breast cancer with accuracy of 99.6% is achieved, and the accuracy is enormously improved.

Further, according to the affection determination technique using top 100 types of feature miRNAs extracted by the affection determination device according to the present invention using 2500 types of miRNAs, diagnosis of breast cancer is possible with accuracy of 99.57%, and the affection determination can be made with accuracy comparable to the case of using 2500 types of miRNA.

### [Second Embodiment]

In the first embodiment, the description has been made using calculation to obtain the loss function Lᵢ as calculation for disease affection determination, and the gradient of each feature of the loss function Lᵢ as the degree of importance for feature extraction. However, the present invention is not limited to this example, and other examples will be described in a second embodiment.

In the second embodiment, a linear classifier is learned by local interpretable model-agnostic explanations (LIME), and the degree of importance is obtained in the process of learning. The learning is performed to obtain training data as an input and a linear classifier as a learned model as an output. For each training data, a linear learner that approximates a trained predictor is learned. In this case, noise is added to the sample data to create a plurality of artificial feature vectors, and the artificial feature vector is given to the trained predictor to obtain a virtual label (or probability distribution on the label). The linear classifier is learned using the obtained artificial feature vector and the virtual label. The linear classifier for a label y obtained in this manner can be expressed as fᵢ(y|x) = ∑ⱼwᵢⱼxⱼ. From this linear classifier, the degree of importance Sⱼ is calculated. For example, the degree of importance Sⱼ is calculated as Sⱼ = |∑ᵢwᵢⱼ|. Ranking is performed on the basis of the degree of importance Sⱼ obtained in this manner, and feature biomarkers regarding the disease are extracted.

As described above, even if the degree of importance is calculated using the technique of learning the linear classifier by LIME, affection determination can be performed with accuracy and the feature biomarkers can be extracted.

### [Third Embodiment]

Calculation for feature extraction may be obtaining the degree of importance of each feature by calculation by layer-wise relevance propagation (LRP). However, in this technique, assumems that a predictor has following three properties: (1) having a neural network without branching; (2) having layers with different dimensions in input/output of dimensions, of the layers in the neural network used for the predictor, being all binding layers only; and (3) outputting k-dimensional vector corresponding to the number k of types of labels, and an i-th output representing i-th prediction probability.

The degree of importance Sij is calculated for each sample data i and each feature j. In the calculation, first, a feature of the sample data i is provided to a trained neural network and forward propagation is performed. The layers are crossed in reverse order from the output unit and an importance vector R representing the degree of importance in each layer is recursively calculated. The order of proceeding in the calculation is similar to an error back propagation method, but calculation actually performed in each layer is different. A j-th value of the importance vector R at the input unit (which has the same dimension as the input feature vector, similarly to the error back propagation method) is defined as the importance Sij for the feature j. After the calculation is completed for all the sample data, the degree of importance Sⱼ of each feature j is calculated, for example, like Sⱼ = |∑ᵢSᵢⱼ|. Ranking is performed on the basis of the degree of importance Sⱼ obtained in this manner, and feature biomarkers regarding a disease are extracted.

As described above, even if the degree of importance is calculated using the technique of learning the predictor by LRP, affection determination can be performed with accuracy and the feature biomarkers can be extracted.

In the first to third embodiments, the examples using the miRNAs as the biomarkers have been described. However, anything can be the biomarkers as long as expression levels thereof can be detected and quantified in a human-derived sample. The greatest feature of the present invention is that the biomarkers can be used in the affection determination without recognizing what biomarker acts on a disease, and thus not only the miRNA but also a quantifiable biomarker can be employed without any problem.

In the first to third embodiments, calculation to obtain the absolute value of the sum of the degrees of importance of the plurality of sample data has been performed for each feature corresponding to the biomarker, as the calculation to extract the feature biomarker, but the present invention is not limited thereto. For example, maximum values of the degree of importance in a plurality of sample data are extracted for each feature corresponding to a biomarker, as the degrees of importance of the feature, the degrees of importance (maximum values) of each extracted feature are compared, a predetermined number of biomarkers from the top in descending order of the value of the degree of importance are extracted as the feature biomarkers regarding the disease.

The affection determination and the feature extraction by the disease affection determination device 10 described in the first to third embodiments are applicable not only to the exemplified breast cancer but also to diagnosis of various cancers, and are also applicable to various diseases other than cancer.

### [Fourth Embodiment]

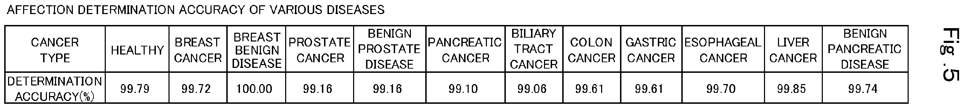

As described in the first embodiment, the present invention is applicable to affection determination of various diseases. Fig. 5 is a table illustrating affection determination accuracy of when the present invention is applied for various diseases. Fig. 5 illustrates a result of a case where machine learning is performed from sample data of patients affected with diseases and healthy subjects, and affection determination is performed using a learned model that enables affection determination in a plurality of cancer types. Here, as an example, a case of using a plurality of sample data of patients affected with a specific cancer type and a plurality of sample data of healthy subjects, as sample data for learning, will be described. Here, the sample data of a patient affected with a specific cancer type is, for example, "sample data of a patient affected with breast cancer", "sample data of a patient affected with prostate cancer", or the like, and a label of one cancer type is attached to one sample data. Here, a plurality of cancer types such as breast cancer and prostate cancer is determined in advance as a group of diseases, and to determine whether affected with any disease in the group of diseases or whether not affected with any of the diseases determined in the group of diseases, the sample data of the patient affected with a disease determined in the group of diseases and the sample data of a patient not affected with any of the diseases determined in the group of diseases are used.

A patient not affected with any of the diseases determined in the disease group is treated as a healthy subject. In this case, a label indicating a cancer type is not provided, and a label indicating a health subject is provided instead. (In a case where the label indicating a health subject is not separately provided and the label indicating a cancer type is not provided, the sample data may be determined to be sample data of a healthy subject. However, to simplify description, the label indicating a healthy subject is provided instead, without providing the label indicating a cancer type.)

As a result of the machine learning, when the affection determination of the sample data of a specific patient is performed using the obtained learned model, presence of affection of a plurality of cancers such as "presence of affection of breast cancer, presence of affection of prostate cancer, presence of affection of pancreatic cancer ..." is independently and exclusively determined, and presence of affection is determined for one of the cancer types. For example, for the following three cancers, determination is made such as "the affection rate of breast cancer being 70%, the affection rate of prostate cancer being 20%, the affection rate of prostate cancer being 10%, the probability of being a healthy subject being 0%". Then, for this patient, a result of determination that the patient is affected with breast cancer with the highest affection rate is output. Meanwhile, in a case where the determination is made such as "the affection rate of breast cancer being 10%, the affection rate of prostate cancer being 5%, the affection rate of prostate cancer being 5%, and the probability of being a healthy subject being 80%". The patient is determined to be a healthy person with the highest probability. Such a technique is generally called multi-class, and when the above determination results are summed up, it becomes 100%. Fig. 5 is a list that summarizes the determination accuracy for cancer types and benign diseases by such a method. Note that details of benign diseases and malignant diseases will be described below.

The total number of samples used for the determination in Fig. 5 is about 5000. As illustrated in Fig. 5, the determination accuracy for healthy subject is 99.79%, the determination accuracy for breast cancer is 99.72%, the determination accuracy for breast benign disease is 100%, the determination accuracy for prostate cancer is 99.16%, the determination accuracy for benign prostate disease is 99.16%, the determination accuracy for pancreatic cancer is 99.10%, the determination accuracy for biliary tract cancer is 99.06%, the determination accuracy for colon cancer is 99.61%, the determination accuracy for gastric cancer is 99.61 %, the determination accuracy for esophageal cancer is 99.70%, the determination accuracy for liver cancer is 99.85%, the determination accuracy for benign pancreatic disease is 99.74%, and the affection determination for various diseases can be performed with very high accuracy.

Furthermore, as a feature of the present invention, affection determination can be performed not only for malignant diseases but also for benign diseases. As illustrated in Fig. 5, the relationships between breast cancer and breast benign disease, between prostate cancer and benign prostate disease, among pancreatic cancer and biliary tract cancer, and benign pancreatic disease are in the relationship between a malignant disease and a benign disease. That is, if learning is performed for a plurality of diseases in the relationship between a malignant disease and a benign disease in the disease affection determination device, and these relationships are simultaneously determined, there is an effect to be able to determine whether the disease is a malignant disease or a benign disease. For example, a learned model in which both breast cancer and breast benign disease are determinable is generated using a plurality of training data to which label information as to whether affected with respective diseases is attached so that both breast cancer and breast benign disease can be determined. If affection determination is performed using this learned model, breast cancer and breast benign disease can be distinguished and determined with high accuracy. By the process, malignancy and benignancy can be accurately distinguished. For example, in breast cancer, it has been very difficult to distinguish between malignancy and benignancy by any conventional diagnostic method, especially it has been impossible at an early stage. Therefore, there is a problem that breasts may be resected even if there is a possibility of benignancy. However, according to the disease affection determination of the present invention, malignancy and benignancy are distinguished, thereby to perform appropriate treatment without resecting portions having benign possibilities. In this respect, it can be said that the influence on patient's QOL is enormous and this is a breakthrough invention.

To realize an affection determination device for performing affection determination of a plurality of diseases at the same time, a plurality of sample data to which label information indicating affection of any of the plurality of diseases is attached is prepared as the training data for generating the learned model. For example, as illustrated in Fig. 5, to generate a learned model for performing affection determination at the same time for a total of twelve types including eleven types of diseases and one type indicating health subject including healthy, breast cancer, breast benign disease, prostate cancer, benign prostate disease, pancreatic cancer, biliary tract cancer, colon cancer, gastric cancer, esophageal cancer, liver cancer, benign pancreatic disease, a plurality of sample data of patients affected with any of the eleven types of diseases and sample data to which label information about the eleven diseases is attached is prepared. Further, a plurality of sample data of healthy patients in which label information is attached only to the label item for healthy subject unaffected with the eleven diseases is also prepared. Assuming that the label information is expressed by flags of "0" and "1", in the sample data of the patient affected with breast cancer, "1" is set only to the label item of breast cancer and "0" is set to all label items of the other 10 diseases.

Learning is performed to be able to output an affection determination result that is the same as the label information, using the plurality of sample data to which label information of the eleven types of diseases is attached and the plurality of sample data of healthy subjects in which the label information is attached only to the label item for healthy subject unaffected with the eleven types of diseases prepared as described above, to obtain the learned model. In the learning process, in the case of a neural network, multitask learning such as sharing a lower layer (layer close to the input) of the neural network by individual tasks may be performed. With the multitask learning, knowledge obtained in individual prediction tasks can be shared among the tasks, and improvement of accuracy can be expected.

Note that the learned model is not limited to the case of performing the affection determination for all the eleven types at the same time, and the learned model may be a learned model in which the affection of only two types of breast cancer and breast benign disease is determinable, a learned model in which the affection of only two types of prostate cancer and benign prostate disease is determinable, a learned model in which the affection of three types of pancreatic cancer, biliary tract cancer, and benign pancreatic disease is determinable, or a learned model in which the affection of a larger number of diseases than the eleven diseases is determinable at the same time.

Furthermore, in the above description of the embodiment, a plurality of sample data to which label information indicating affection of any one of a plurality of diseases is attached has been prepared as the training data for generating a learned model, and in that case, the affection determination has been performed on the assumption that the patient is affected with only a specific type of the plurality of cancer types or the patient is not affected with any of the plurality of cancer types. However, there are cases where a patient is affected with a plurality of cancer types due to metastatic cancer or the like. In this case, affection determination can be performed by modifying the way of making the label of the sample data to be used as the training data, and applying a technique similar to the above-described embodiment. As an example, in a case where a patient is affected with lung cancer and gastric cancer, training sample data having label items corresponding to lung cancer and gastric cancer, which are set to "1" and other label items that are set to "0" is prepared and a learned model is created by machine learning, and affection determination is performed using the learned model. These techniques are called multi-labeling, and has an effect to perform the affection determination for one or more cancers by a single determination, by attaching labels indicating a plurality of different cancer diseases to the training sample data and creating a learned model by performing machine learning.

With the affection determination device using the learned model obtained as described above, the affection determination of malignant diseases and benign diseases can be performed at the same time, or the affection determination of a plurality of diseases can be performed at the same time in a single examination.

### [Fifth Embodiment]

Although the affection determination device in the first to fourth embodiments can output conclusions as to whether a patient is affected with a disease by inputting sample data of the patient to the learned model, biomarkers that influence the determination to reach the conclusion cannot be obtained. However, there is a possibility of arising of needs to know which biomarkers influence the determination in order to recognize the reason why the conclusion is led by a doctor or to explain the reason why the conclusion is led to a patient by a doctor.

Therefore, in inputting sample data of a patient to be determined for affection to the learned model and performing affection determination, the degree of importance of each feature dimension corresponding to a biomarker may be calculated, and a biomarker having contributed to the conclusion of the affection determination may be extracted and output on the basis of the magnitude of the value of the degree of importance.

The degree of importance of each feature dimension corresponding to a biomarker is calculated as a gradient gⱼ regarding a feature xⱼ, by a process of calculating a loss function L, using the learned model, for the sample data, and a process of performing error back propagation with a value L of the loss function as a starting point and calculating a gradient gⱼ = ∂L/∂xⱼ for the feature xⱼ corresponding to each of a plurality of types of biomarkers. Calculation of the gradient here is similar to that of the first embodiment. However, the gradient here is different from the first embodiment in that the gradient is calculated for only the sample data of one patient, instead of calculating a sum of a plurality of sample data.

Further, the degree of importance may be calculated by learning a linear classifier by local interpretable model-agnostic explanations (LIME), and the degree of importance is obtained in the process of learning. As described in the second embodiment, the linear classifier for a label y obtained by performing learning by LIME can be expressed as fᵢ(y|x) = ∑ⱼwᵢⱼxⱼ. In a case where there is one sample data of a patient to be determined for affection, i for the number of samples is one, and thus the degree of importance for the feature xⱼ can be calculated by wⱼ. That is, a linear learner that approximates the learned model in the affection determination unit 12 is learned by LIME, and a coefficient of the linear learner corresponding to a feature dimension of each biomarker of a case where the sample data of the patient to be determined for affection is input to the linear learner is obtained as the degree of importance of each biomarker.

Further, for calculation of the degree of importance, the degree of importance of each feature may be obtained by calculation by layer-wise relevance propagation (LRP), for example. As described in the third embodiment, in the calculation by the LRP, the feature of the sample data of the patient to be determined for affection is provided to the trained neural network and forward propagation is performed. Layers are crossed in reverse order from the output unit, and the importance vector R that represents the degree of importance in each layer is recursively calculated, whereby the importance vector R can be calculated as the degree of importance of each feature dimension feature to a biomarker.

The above-described three methods of calculating the degree of importance are examples, and other methods can be employed as long as methods can calculate the degree of importance for each biomarker of the sample data of the patient to be determined for affection.

As described above, the degree of importance is calculated for each biomarker of the sample data of the patient to be determined for affection, the biomarker having contributed to the conclusion of the affection determination is extracted on the basis of the calculated degree of importance, and the marker is output from a determination contribution biomarker output unit. Extraction of biomarkers having contributed to the conclusion may be performed by outputting a predetermined number of biomarkers from the top in descending order of the value of the degree of importance, or employment of a method of displaying a heat map, or the like can be considered.

In this way, the biomarker having contributed to the conclusion is output from the determination contribution biomarker output unit together with the affection determination result, whereby which biomarker has contributed to the affection determination can be presented to each individual patient, and thus the biomarker can be described as the ground for determination when a doctor conveys the affection determination result to the patient. Further, the doctor can recognize the reason why the conclusion is led. Furthermore, by knowing the biomarker that is the ground for affection determination, there is also a possibility of use in a method of individually selecting a treatment method according to the biomarker having contributed to the determination in the future.

### [Sixth Embodiment]

In the first to third embodiments, the calculation method based on gradient calculation, LIME, LRP, and the like has been described as the method of calculating the degree of importance in the feature extraction unit 13, and the degree of importance has been calculated by obtaining the absolute value of the sum of the plurality of sample data. However, the calculation method is not limited to the calculation method based on the absolute value of the sum. For example, the degree of importance may be calculated by employing a calculation method of an L¹ norm, an L² norm, an L^{P} norm that is generalization of the aforementioned norms, and the like.

That is, in a disease feature extraction device provided with a sample data acquisition unit configured to acquire sample data in which respective expression levels of biomarkers including a plurality of types of miRNAs in a human-derived sample are recorded for each individual, an affection determination unit including a learned model in which affection of diseases is determinable obtained in advance by performing machine learning using training data, and a feature extraction unit configured to input a plurality of sample data to which label information of disease affection is attached, to the affection determination unit to determine affection, to obtain the degrees of importance of respective feature of a plurality of biomarkers obtained with the learned model by affection determination calculation, for each sample data, and to extract a predetermined number of biomarkers as feature biomarkers regarding the disease on the basis of numerical values of the degree of importance of the plurality of sample data, for each biomarker, a process of extracting a predetermined number of biomarkers important in disease affection determination in descending order on the basis of the magnitude of the degree of importance, for example, top 100 biomarkers becomes possible by employing not only the absolute value of the sum but also the calculation method of an L¹ norm, an L² norm, and an L^{P} norm that is generalization of the aforementioned norms, as the method of calculating the degree of importance on the basis of gradient calculation, LIME, LRP, or the like in the feature extraction unit.

As advantages of extracting important biomarkers in the disease affection determination, an effect to find a biomarker specific to a disease by extracting a feature biomarker of each disease and performing comparison among the plurality of diseases can be expected, and an effect to become a trigger to find an unknown relevancy between a feature biomarker and a disease can be expected, in addition to the effect to decrease the processing capacity required for a computer and improve the processing speed while maintaining accuracy of the affection determination described in the first embodiment.

### [Seventh Embodiment]

In the first to sixth embodiments, the description has been made using the example of employing the neural network as the machine learner that configures the learned model, but the machine leaner is not limited to the neural network and various techniques such as gradient boosting, random forest (decision forest), extra tree, support vector machine, logistic regression, or K neighborhood method can be employed as the machine learner. In the machine learner other than the neural network, the error back propagation method cannot be applied when calculating the degree of importance. Therefore, in such a case, the degree of importance can be calculated by calculating a gradient by numerical differentiation.

### [Eighth Embodiment]

In the first to seventh embodiments, the configuration to input the sample data of the patient to be determined for affection to the disease affection determination device composed of one learned model, and perform the affection determination in the affection determination unit 12 composed of learned model has been described. However, the present invention is not limited to these examples. Prediction of affection determination may be performed by each of a plurality of machine learners, and an affection determination result may be obtained by a stacking machine learner that outputs a determination result on the basis of the plurality of obtained prediction results.

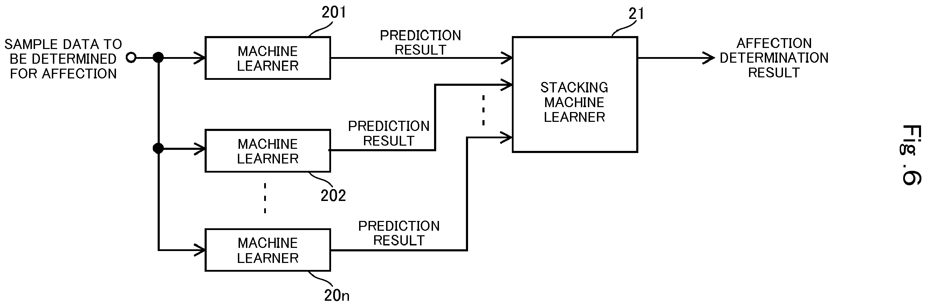

Fig. 6 is a block diagram illustrating a configuration of a disease affection determination device 22 that employs a stacking technique. In Fig. 6, machine learners 201, 202, ..., and 20n are different types of machine learners. Types of the machine learners 201, 202, ..., and 20n include neural network, gradient boosting, random forest (decision forest), extra tree, support vector machine, logistic regression, K neighborhood method, and the like. Further, the machine learner may differently use Feedforward, CNN, VAE, GAN, AAE and the like that are neural networks. The machine learners 201, 202, ..., and 20n are configured from a learned model which has learned in advance affection determination for the same disease on the basis of the same training data. To employ a stacking technique, at least two or more machine learners of different types need to be used.

The stacking machine learner 21 is configured from a learned model that has learned in advance to output a final affection determination result about the sample data of the patient to be determined for affection, using a plurality of prediction results output from the respective machine learners 201, 202,..., and 20n. The stacking machine learner 21 may be any of the neural network, gradient boosting, random forest (decision forest), extra tree, support vector machine, logistic regression, K nearest neighbor method, and the like.

As illustrated in Fig. 6, a disease affection determination device 22 that employs the stacking technique first inputs sample data of a patient to be determined for affection to each of the plurality of machine learners 201, 202, ..., and 20n. Each of the plurality of machine learners 201, 202, ..., and 20n outputs a prediction result as to whether affected with the disease on the basis of each learned model. The plurality of prediction results is input to the stacking machine learner 21. The stacking machine learner 21 outputs a final affection determination result on the basis of the plurality of prediction results.

As described above, by use of the disease affection determination device 22 that employs the stacking technique, determination accuracy can be improved as compared with affection determination by a single machine learner. That is because machine learners have possibility of having strong and weak points in grasping feature of sample data depending on the types of the machine learners. In contrast, according to the affection determination device 22 that employs the stacking, the stacking machine learner 21 learns interaction and strong and weak points of the respective machine learners, and thus final affection determination reflecting the interaction and the strong and weak points can be performed, whereby the determination accuracy can be improved as compared with the case of a single machine learner, accordingly.

### [Ninth Embodiment]

In the first to seventh embodiments, the description about the disease affection determination device including one machine learner has been made. However, ensemble learning using prediction results respectively predicted by a plurality of machine learners may be performed. The ensemble learning is a technique of obtaining a geometric mean of prediction probabilities respectively output by a plurality of machine learners and outputting a final prediction result. The plurality of machine learners may be of the same type or machine learners of different types may be employed. By performing such ensemble learning, the affection determination accuracy of diseases can be improved. In addition, the ensemble learning can be applied in the disease affection determination device 22 that employs the stacking technique described in the eighth embodiment. In this case, a plurality of the stacking machine learners 21 is prepared, the geometric mean of outputs of prediction results of the plurality of stacking machine learners 21 is obtained, and the final prediction result is output, whereby the affection determination accuracy of diseases can be improved.

In the above description of the embodiment, the description by the miRNAs in the human-derived reagent has been made as a representative of organisms, but it is needless to say that a person having ordinary knowledge in the field to which the invention belongs can improve the affection determination accuracy of similar diseases by use of a similar technique to the present embodiment in organisms other than human beings, such as animals including pets and livestock.

### [Appendix]

The above-described embodiment has been described such that a person having ordinary knowledge in the field to which the invention belongs can carry out the invention.
[1] A disease affection determination device including:
   a sample data acquisition unit configured to acquire sample data including respective expression levels of biomarkers including a plurality of types of miRNAs in an individual organism-derived sample;
   a learned model in which affection of diseases is determinable obtained in advance by performing machine learning using training data; and
   an affection determination unit configured to perform affection determination for the sample data on the basis of the degree of importance of each biomarker, using the learned model.
[2] A disease affection determination device including:
   a sample data acquisition unit configured to acquire sample data including respective expression levels of biomarkers including a plurality of types of miRNAs in an individual organism-derived sample;
   a learned model in which affection of diseases is determinable, obtained in advance by performing machine learning using training data;
   an importance calculation unit configured to input the sample data to the learned model to quantify the degree of importance of each biomarker; and
   an affection determination unit configured to perform affection determination for the sample data from the degree of importance.
[3] The disease affection determination device according to [1] or [2], including:
   a feature extraction unit configured to extract a feature biomarker regarding the disease on the basis of the degree of importance, wherein
   the affection determination is performed on the basis of feature importance that is the degree of importance of each feature biomarker in a case of performing disease determination only with the extracted feature biomarker.
[4] The disease affection determination device according to [1] or [2], including:
   a feature extraction unit configured to extract a feature biomarker regarding the disease on the basis of the degree of importance; and
   a feature importance calculation unit configured to quantify feature importance that is the degree of importance of each feature biomarker in a case of performing disease determination only with the extracted feature biomarker, wherein
   the affection determination unit performs the affection determination from the feature importance.
[5] The disease affection determination device according to any one of [2] to [4], wherein
   the importance calculation unit quantifies the degrees of importance of features of respective biomarkers by a process of calculating a loss function Lᵢ regarding the i-th sample data, using the learned model, for each sample data, a process of performing error back propagation with a value Lᵢ of the loss function as a starting point and calculating a gradient gᵢⱼ = ∂Lᵢ/∂xⱼ regarding a feature xⱼ corresponding to each of a plurality of types of biomarkers of the sample i, and a process of obtaining an absolute value of a sum of gradients about all the samples as the degree of importance Sⱼ = |∑_{i}gᵢⱼ| of the feature.
[6] The disease affection determination device according to any one of [1] to [5], wherein
   the training data is the sample data to which label information as to whether individuals are affected with diseases is attached.
[7] The disease affection determination device according to [6], wherein
   generation of the learned model is performed after a whitening process is performed, the whitening process being of linear transformation of each dimension such that an average over the entire training data becomes 0 and thevariance becomes 1, for each dimension of a feature vector of the training data.
[8] A disease affection determination method including the steps of:
   acquiring sample data including respective expression levels of biomarkers including a plurality of types of miRNAs in an individual organism-derived sample;
   generating a learned model in which affection of diseases is determinable, obtained in advance by performing machine learning using training data; and
   performing affection determination for the sample data on the basis of the degree of importance of each biomarker, using the learned model.
[9] A disease affection determination method including the steps of:
   acquiring sample data including respective expression levels of biomarkers including a plurality of types of miRNAs in an individual organism-derived sample;
   generating a learned model in which affection of diseases is determinable, obtained in advance by performing machine learning using training data;
   inputting the sample data to the learned model to quantify the degree of importance of each biomarker; and
   performing affection determination for the sample data from the degree of importance.
[10] The disease affection determination method according to [8] or [9], including the step of:
   extracting a feature biomarker regarding the disease on the basis of a sum of the degrees of importance, wherein
   the affection determination is performed on the basis of feature importance that is the degree of importance of each feature biomarker in a case of performing disease determination only with the extracted feature biomarker.
[11] The disease affection determination method according to [8] or [9], including the steps of:
   extracting a feature biomarker regarding the disease on the basis of the sum of the degrees of importance; and
   quantifying feature importance that is the degree of importance of each feature biomarker in a case of performing disease determination only with the extracted feature biomarker, wherein
   the affection determination is performed from the feature importance in the step of performing affection determination.
[12] The disease affection determination method according to any one of [9] to [11], wherein,
   in the step of calculating the degree of importance, the degrees of importance of features of respective biomarkers are quantified by a process of calculating a loss function Lᵢ regarding the i-th sample data, using the learned model, for each sample data, a process of performing error back propagation with a value Li of the loss function as a starting point and calculating a gradient gᵢⱼ = ∂Lᵢ/∂xⱼ regarding a feature xⱼ corresponding to each of a plurality of types of biomarkers of the sample i, and a process of obtaining an absolute value of a sum of gradients about all the samples as the degree of importance Sⱼ = |∑_{i}gᵢⱼ| of the feature.
[13] The disease affection determination method according to any one of [8] to [12], wherein
   the training data is the sample data to which label information as to whether individuals are affected with diseases is attached.
[14] The disease affection determination method according to [12], wherein
   generation of the learned model is performed after a whitening process is performed, the whitening process being of linear transformation of each dimension such that an average over the entire training data becomes 0 and the variance becomes 1, for each dimension of a feature vector of the training data.
[15] A disease feature extraction device including:
   a sample data acquisition unit configured to acquire sample data in which respective expression levels of biomarkers including a plurality of types of miRNAs in an individual organism-derived sample are recorded for each individual;
   an affection determination unit including a learned model in which affection of diseases is determinable, obtained in advance by performing machine learning using training data; and
   a feature extraction unit configured to input a plurality of sample data to which label information of disease affection is attached, to the affection determination unit to determine affection, to quantify the degrees of importance of respective feature of a plurality of biomarkers obtained with the learned model by affection determination calculation, for each sample data, and to extract a predetermined number of biomarkers as feature biomarkers regarding the disease on the basis of numerical values of the degree of importance of the plurality of sample data, for each biomarker.
[16] The disease feature extraction device according to [15], wherein the feature extraction unit quantifies the degree of importance of features of respective biomarkers by a process of calculating a loss function Lᵢ regarding the i-th sample data, using the learned model, for each sample data, a process of performing error back propagation with a value Lᵢ of the loss function as a starting point and calculating a gradient gᵢⱼ = ∂Lᵢ/∂xⱼ regarding a feature xⱼ corresponding to each of a plurality of types of biomarkers of the sample i, and a process of obtaining an absolute value of a sum of gradients about all the samples as the degree of importance Sⱼ = |∑_{i}gᵢⱼ| of the feature.
[17] The disease feature extraction device according to any one of [15] to [16], wherein
   the training data is the sample data to which label information as to whether individuals are affected with diseases is attached.
[18] The disease feature extraction device according to any one of [15] to [17], wherein
   generation of the learned model is performed after a whitening process is performed, the whitening process being of linear transformation of each dimension such that an average over the entire training data becomes 0 and the variance becomes 1, for each dimension of a feature vector of the training data.
[19] The disease feature extraction device according to [18], wherein
   the plurality of sample data to which label information of disease affection is attached, which is used in the feature extraction unit, is used after a whitening process is performed, the whitening process being of linear transformation of each dimension such that an average over the entire sample data becomes 0 and the variance becomes 1, for each dimension of a feature vector.
[20] A disease feature extraction method including the steps of:
   acquiring sample data in which respective expression levels of biomarkers including a plurality of types of miRNAs in an individual organism-derived sample are recorded for each individual;
   generating a learned model in which affection of diseases is determinable, obtained in advance by performing machine learning using training data; and
   inputting a plurality of sample data to which label information of disease affection is attached, to the learned model to determine affection, quantifying the degrees of importance of respective feature of a plurality of biomarkers obtained with the learned model by affection determination calculation, for each sample data, and extracting a predetermined number of biomarkers as feature biomarkers regarding the disease on the basis of numerical values of the degree of importance of the plurality of sample data, for each biomarker.
[21] A disease affection determination device including:
   a sample data acquisition unit configured to acquire sample data including respective expression levels of biomarkers including a plurality of types of miRNAs in an individual organism-derived sample;
   a learned model in which affection of diseases is determinable, obtained in advance by performing machine learning using a plurality of training data including sample data each including items for identifying presence or absence of affection of a plurality of diseases and to which label information is attached, the label information indicating whether individuals are affected with any of the diseases; and
   an affection determination unit configured to perform affection determination as to whether sample data to be determined is affected with a plurality of diseases, using the learned model.
[22] The disease affection determination device according to [21], further including:
   a determination contribution biomarker output unit configured to extract a biomarker that has contributed to a disease affection determination result, of the biomarkers included in the sample data to be determined for disease affection, and output the extracted biomarker.
[23] The disease affection determination device according to [22], wherein
   the determination contribution biomarker output unit calculates, by a process of calculating a loss function L, using the learned model, for the sample data, and a process of performing error back propagation with a value L of the loss function as a starting point and calculating a gradient gⱼ = ∂L/∂xⱼ for a feature xⱼ corresponding to each of a plurality of types of biomarkers, the degree of importance of each feature dimension corresponding to the biomarker as the gradient gⱼ for the feature xⱼ, and extracts a predetermined number of biomarkers as the biomarkers that have contributed to the disease affection determination result on the basis of the magnitude of the degree of importance.
[24] The disease affection determination device according to [22], wherein
   the determination contribution biomarker output unit learns a linear learner that approximates the learned model in the affection determination unit by LIME, calculates a coefficient of the linear leaner, the coefficient corresponding to the feature dimension of each biomarker of when the sample data to be determined for affection is input to the linear learner, as the degree of importance of each biomarker, and extracts a predetermined number of biomarkers as the biomarkers that have contributed to the disease affection determination result on the basis of the magnitude of the degree of importance.
[25] The disease affection determination device according to [22], wherein
   the determination contribution biomarker output unit performs forward propagation by providing a feature of sample data of a patient to be determined for affection to the learned model in the affection determination unit by LRP, recursively calculates an importance vector R representing the degree of importance in each layer, crossing layers in reverse order from the output unit, calculates the importance vector R as the degree of importance of each feature dimension corresponding to each biomarker, and extracts a predetermined number of biomarkers as the biomarkers that have contributed to the disease affection determination result on the basis of the magnitude of the degree of importance.
[26] A disease affection determination device including:
   a sample data acquisition unit configured to acquire sample data including respective expression levels of biomarkers including a plurality of types of miRNAs in an individual organism-derived sample;
   at least two or more machine learners configured to perform machine learning commonly using a plurality of training data including sample data each including items for identifying presence or absence of affection of a plurality of diseases and to which label information is attached, the label information indicating whether individuals are affected with any of the diseases, the machine learners respectively including different types of learned models that have learned in advance to determine affection of the same disease, the machine learners configured to output a prediction result as to whether sample data to be determined for disease affection has affected a disease; and
   a stacking machine learner that has learned in advance to output a final determination result, using the prediction results from the plurality of machine learners as inputs, and configured to output a determination result as to whether the sample data to be determined for affection is affected with a disease on the basis of the prediction results from the plurality of machine learners.
[27] The disease affection determination device according to any one of [21] to [26], wherein
   the plurality of diseases includes at least two types of breast cancer, breast benign disease, prostate cancer, benign prostate disease, pancreatic cancer, biliary tract cancer, colon cancer, gastric cancer, esophageal cancer, liver cancer, and benign pancreatic disease.
[28] A disease affection determination device including:
   a plurality of sample data respectively acquired from individual organisms and including respective expression levels of a plurality of types of biomarkers including miRNA in individual organism-derived samples;
   a learned model in which presence or absence of affection of a plurality of diseases is determinable, the plurality of diseases being output as a result of machine learning using, as training data, sample data with label information in which items for identifying whether each individual organism has affected the plurality of diseases are provided as label information, for each of the plurality of sample data; and
   an affection determination unit configured to determine presence or absence of affection of each of the plurality of diseases, using the learned model, for sample data newly acquired from another organism for which affection determination is to be performed.
[29] A disease affection determination device including:
   a plurality of sample data respectively acquired from individual organisms and including respective expression levels of a plurality of types of biomarkers including miRNA in an individual organism-derived sample;
   a learned model in which presence or absence of affection of a predetermined disease is determinable, the predetermined disease being output as a result of machine learning using, as training data, sample data with label information in which items for identifying whether each individual organism is affected with any one of a predetermined group of diseases determined in advance or whether each individual organism is not affected with any of the predetermined group of diseases determined in advance, as information regarding the disease when affected with the disease or information indicating that the individual organism is not affected when not affected, as label information for each of the plurality of sample data; and
   an affection determination unit configured to determine whether affected with any one of the predetermined group of diseases or whether not affected with any of the predetermined group of diseases, using the learned model, for sample data newly acquired from another organism for which affection determination is to be performed.
[30] A disease affection determination method including the steps of:
   acquiring sample data including respective expression levels of biomarkers including a plurality of types of miRNAs in an individual organism-derived sample;
   generating a learned model in which whether affected with a plurality of diseases is determinable obtained in advance by performing machine learning using a plurality of training data including sample data each including items for identifying presence or absence of affection of a plurality of diseases and to which label information is attached, the label information indicating whether individuals are affected with any of the diseases; and
   performing affection determination as to whether sample data to be determined is affected with a plurality of diseases, using the learned model.
[31] A disease affection determination method including the steps of:
   acquiring sample data including respective expression levels of biomarkers including a plurality of types of miRNAs in an individual organism-derived sample;
   acquiring a plurality of prediction results on the basis of at least two or more machine learners configured to perform machine learning commonly using a plurality of training data including sample data each including items for identifying presence or absence of affection of a plurality of diseases and to which label information is attached, the label information indicating whether individuals are affected with any of the diseases, the machine learners respectively including different types of learned models that have learned in advance to determine affection of the same disease, the machine learners configured to output a prediction result as to whether sample data to be determined for disease affection has affected a disease; and
   acquiring a final determination result on the basis of a stacking machine learner that has learned in advance to output a final determination result, using the prediction results from the plurality of machine learners as inputs, and configured to output a determination result as to whether the sample data to be determined for affection is affected with a disease on the basis of the prediction results from the plurality of machine learners.
[32] A disease affection determination method including the steps of:
   acquiring a plurality of sample data respectively acquired from individual organisms and including respective expression levels of a plurality of types of biomarkers including miRNA in individual organism-derived samples;
   generating a learned model in which presence or absence of affection of a plurality of diseases is determinable, the plurality of diseases being output as a result of machine learning using, as training data, sample data with label information in which items for identifying whether each individual organism has affected the plurality of diseases are provided as label information, for each of the plurality of sample data; and
   determining presence or absence of affection of each of the plurality of diseases, using the learned model, for sample data newly acquired from another organism for which affection determination is to be performed.
[33] A disease affection determination method including the steps of:
   acquiring a plurality of sample data respectively acquired from individual organisms and including respective expression levels of a plurality of types of biomarkers including miRNA in an individual organism-derived sample;
   generating a learned model in which presence or absence of affection of a predetermined disease is determinable, the predetermined disease being output as a result of machine learning using, as training data, sample data with label information in which items for identifying whether each individual organism is affected with any one of a predetermined group of diseases determined in advance or whether each individual organism is not affected with any of the predetermined group of diseases determined in advance, as information regarding the disease when affected with the disease or information indicating that the individual organism is not affected when not affected, as label information for each of the plurality of sample data; and
   determining whether affected with any one of the predetermined group of diseases or whether not affected with any of the predetermined group of diseases, using the learned model, for sample data newly acquired from another organism for which affection determination is to be performed.
[34] A disease affection determination program for causing a computer to realize the processes of:
   acquiring sample data including respective expression levels of biomarkers including a plurality of types of miRNAs in an individual organism-derived sample;
   generating a learned model in which whether affected with a plurality of diseases is determinable obtained in advance by performing machine learning using a plurality of training data including sample data each including items for identifying presence or absence of affection of a plurality of diseases and to which label information is attached, the label information indicating whether individuals are affected with any of the diseases; and
   performing affection determination as to whether sample data to be determined is affected with a plurality of diseases, using the learned model.
[35] A disease affection determination program for causing a computer to realize the processes of:
   acquiring sample data including respective expression levels of biomarkers including a plurality of types of miRNAs in an individual organism-derived sample;
   acquiring a plurality of prediction results on the basis of at least two or more machine learners configured to perform machine learning commonly using a plurality of training data including sample data each including items for identifying presence or absence of affection of a plurality of diseases and to which label information is attached, the label information indicating whether individuals are affected with any of the diseases, the machine learners respectively including different types of learned models that have learned in advance to determine affection of the same disease, the machine learners configured to output a prediction result as to whether sample data to be determined for disease affection has affected a disease; and
   acquiring a final determination result on the basis of a stacking machine learner that has learned in advance to output a final determination result, using the prediction results from the plurality of machine learners as inputs, and configured to output a determination result as to whether the sample data to be determined for affection is affected with a disease on the basis of the prediction results from the plurality of machine learners.
[36] A disease affection determination program for causing a computer to realize the processes of:
   acquiring a plurality of sample data respectively acquired from individual organisms and including respective expression levels of a plurality of types of biomarkers including miRNA in individual organism-derived samples;
   generating a learned model in which presence or absence of affection of a plurality of diseases is determinable, the plurality of diseases being output as a result of machine learning using, as training data, sample data with label information in which items for identifying whether each individual organism has affected the plurality of diseases are provided as label information, for each of the plurality of sample data; and
   determining presence or absence of affection of each of the plurality of diseases, using the learned model, for sample data newly acquired from another organism for which affection determination is to be performed.
[37] A disease affection determination program for causing a computer to realize the processes of:
   acquiring a plurality of sample data respectively acquired from individual organisms and including respective expression levels of a plurality of types of biomarkers including miRNA in an individual organism-derived sample;
   generating a learned model in which presence or absence of affection of a predetermined disease is determinable, the predetermined disease being output as a result of machine learning using, as training data, sample data with label information in which items for identifying whether each individual organism is affected with any one of a predetermined group of diseases determined in advance or whether each individual organism is not affected with any of the predetermined group of diseases determined in advance, as information regarding the disease when affected with the disease or information indicating that the individual organism is not affected when not affected, as label information for each of the plurality of sample data; and
   determining whether affected with any one of the predetermined group of diseases or whether not affected with any of the predetermined group of diseases, using the learned model, for sample data newly acquired from another organism for which affection determination is to be performed.

### Reference Signs List

- 10: Disease affection determination device
- 11: Sample data acquisition unit
- 12: Affection determination unit
- 13: Feature extraction unit
- 14: Storage unit
- 15: Sample data
- 16: Training data
- 17: Learned model
- 18: Importance calculation unit
- 19: Feature importance calculation unit
- 201, 202, ..., 20n: Machine learner
- 21: Stacking machine learner
- 22: Disease affection determination device

## Claims

1. A disease affection determination device comprising:
a sample data acquisition unit configured to acquire sample data including respective expression levels of biomarkers including a plurality of types of miRNAs in an individual organism-derived sample;
a learned model in which affection of diseases is determinable, obtained in advance by performing machine learning using a plurality of training data including sample data each including items for identifying presence or absence of affection of a plurality of diseases and to which label information is attached, the label information indicating whether individuals are affected with any of the diseases; and
an affection determination unit configured to perform affection determination as to whether sample data to be determined is affected with a plurality of diseases, using the learned model.

2. The disease affection determination device according to claim 1, comprising:
a determination contribution biomarker output unit configured to extract a biomarker that has contributed to a disease affection determination result, of the biomarkers included in the sample data to be determined for disease affection, and output the extracted biomarker.

3. The disease affection determination device according to claim 2, wherein
the determination contribution biomarker output unit calculates, by a process of calculating a loss function L, using the learned model, for the sample data, and a process of performing error back propagation with a value L of the loss function as a starting point and calculating a gradient gⱼ = ∂L/∂xⱼ for a feature xⱼ corresponding to each of a plurality of types of biomarkers, the degree of importance of each feature dimension corresponding to the biomarker as the gradient gⱼ for the feature xⱼ, and extracts a predetermined number of biomarkers as the biomarkers that have contributed to the disease affection determination result on the basis of the magnitude of the degree of importance.

4. The disease affection determination device according to claim 2, wherein
the determination contribution biomarker output unit learns a linear learner that approximates the learned model in the affection determination unit by LIME, calculates a coefficient of the linear learner, the coefficient corresponding to the feature dimension of each biomarker of when the sample data to be determined for affection is input to the linear learner, as the degree of importance of each biomarker, and extracts a predetermined number of biomarkers as the biomarkers that have contributed to the disease affection determination result on the basis of the magnitude of the degree of importance.

5. The disease affection determination device according to claim 2, wherein
the determination contribution biomarker output unit performs forward propagation by providing a feature of sample data of a patient to be determined for affection to the learned model in the affection determination unit by LRP, recursively calculates an importance vector R representing the degree of importance in each layer, crossing layers in reverse order from the output unit, calculates the importance vector R as the degree of importance of each feature dimension corresponding to each biomarker, and extracts a predetermined number of biomarkers as the biomarkers that have contributed to the disease affection determination result on the basis of the magnitude of the degree of importance.

6. A disease affection determination device comprising:
a sample data acquisition unit configured to acquire sample data including respective expression levels of biomarkers including a plurality of types of miRNAs in an individual organism-derived sample;
at least two or more machine learners configured to perform machine learning commonly using a plurality of training data including sample data each including items for identifying presence or absence of affection of a plurality of diseases and to which label information is attached, the label information indicating whether individuals are affected with any of the diseases, the machine learners respectively including different types of learned models that have learned in advance to determine affection of the same disease, the machine learners configured to output a prediction result as to whether sample data to be determined for disease affection has affected a disease; and
a stacking machine learner that has learned in advance to output a final determination result, using the prediction results from the plurality of machine learners as inputs, and configured to output a determination result as to whether the sample data to be determined for affection is affected with a disease on the basis of the prediction results from the plurality of machine learners.

7. The disease affection determination device according to any one of claims 1 to 6, wherein
the plurality of diseases includes at least two types of breast cancer, breast benign disease, prostate cancer, benign prostate disease, pancreatic cancer, biliary tract cancer, colon cancer, gastric cancer, esophageal cancer, liver cancer, and benign pancreatic disease.

8. A disease affection determination device comprising:
a plurality of sample data respectively acquired from individual organisms and including respective expression levels of a plurality of types of biomarkers including miRNA in individual organism-derived samples;
a learned model in which presence or absence of affection of a plurality of diseases is determinable, the plurality of diseases being output as a result of machine learning using, as training data, sample data with label information in which items for identifying whether each individual organism has affected the plurality of diseases are provided as label information, for each of the plurality of sample data; and
an affection determination unit configured to determine presence or absence of affection of each of the plurality of diseases, using the learned model, for sample data newly acquired from another organism for which affection determination is to be performed.

9. A disease affection determination device comprising:
a plurality of sample data respectively acquired from individual organisms and including respective expression levels of a plurality of types of biomarkers including miRNA in an individual organism-derived sample;
a learned model in which presence or absence of affection of a predetermined disease is determinable, the predetermined disease being output as a result of machine learning using, as training data, sample data with label information in which items for identifying whether each individual organism is affected with any one of a predetermined group of diseases determined in advance or whether each individual organism is not affected with any of the predetermined group of diseases determined in advance, as information regarding the disease when affected with the disease or information indicating that the individual organism is not affected when not affected, as label information for each of the plurality of sample data; and
an affection determination unit configured to determine whether affected with any one of the predetermined group of diseases or whether not affected with any of the predetermined group of diseases, using the learned model, for sample data newly acquired from another organism for which affection determination is to be performed.

10. A disease affection determination method comprising the steps of:
acquiring sample data including respective expression levels of biomarkers including a plurality of types of miRNAs in an individual organism-derived sample;
generating a learned model in which whether affected with a plurality of diseases is determinable obtained in advance by performing machine learning using a plurality of training data including sample data each including items for identifying presence or absence of affection of a plurality of diseases and to which label information is attached, the label information indicating whether individuals are affected with any of the diseases; and
performing affection determination as to whether sample data to be determined is affected with a plurality of diseases, using the learned model.

11. A disease affection determination method comprising the steps of:
acquiring sample data including respective expression levels of biomarkers including a plurality of types of miRNAs in an individual organism-derived sample;
acquiring a plurality of prediction results on the basis of at least two or more machine learners configured to perform machine learning commonly using a plurality of training data including sample data each including items for identifying presence or absence of affection of a plurality of diseases and to which label information is attached, the label information indicating whether individuals are affected with any of the diseases, the machine learners respectively including different types of learned models that have learned in advance to determine affection of the same disease, the machine learners configured to output a prediction result as to whether sample data to be determined for disease affection has affected a disease; and
acquiring a final determination result on the basis of a stacking machine learner that has learned in advance to output a final determination result, using the prediction results from the plurality of machine learners as inputs, and configured to output a determination result as to whether the sample data to be determined for affection is affected with a disease on the basis of the prediction results from the plurality of machine learners.

12. A disease affection determination method comprising the steps of:
acquiring a plurality of sample data respectively acquired from individual organisms and including respective expression levels of a plurality of types of biomarkers including miRNA in individual organism-derived samples;
generating a learned model in which presence or absence of affection of a plurality of diseases is determinable, the plurality of diseases being output as a result of machine learning using, as training data, sample data with label information in which items for identifying whether each individual organism has affected the plurality of diseases are provided as label information, for each of the plurality of sample data; and
determining presence or absence of affection of each of the plurality of diseases, using the learned model, for sample data newly acquired from another organism for which affection determination is to be performed.

13. A disease affection determination method comprising the steps of:
acquiring a plurality of sample data respectively acquired from individual organisms and including respective expression levels of a plurality of types of biomarkers including miRNA in an individual organism-derived sample;
generating a learned model in which presence or absence of affection of a predetermined disease is determinable, the predetermined disease being output as a result of machine learning using, as training data, sample data with label information in which items for identifying whether each individual organism is affected with any one of a predetermined group of diseases determined in advance or whether each individual organism is not affected with any of the predetermined group of diseases determined in advance, as information regarding the disease when affected with the disease or information indicating that the individual organism is not affected when not affected, as label information for each of the plurality of sample data; and
determining whether affected with any one of the predetermined group of diseases or whether not affected with any of the predetermined group of diseases, using the learned model, for sample data newly acquired from another organism for which affection determination is to be performed.

14. A disease affection determination program for causing a computer to realize the processes of:
acquiring sample data including respective expression levels of biomarkers including a plurality of types of miRNAs in an individual organism-derived sample;
generating a learned model in which whether affected with a plurality of diseases is determinable obtained in advance by performing machine learning using a plurality of training data including sample data each including items for identifying presence or absence of affection of a plurality of diseases and to which label information is attached, the label information indicating whether individuals are affected with any of the diseases; and
performing affection determination as to whether sample data to be determined is affected with a plurality of diseases, using the learned model.

15. A disease affection determination program for causing a computer to realize the processes of:
acquiring sample data including respective expression levels of biomarkers including a plurality of types of miRNAs in an individual organism-derived sample;
acquiring a plurality of prediction results on the basis of at least two or more machine learners configured to perform machine learning commonly using a plurality of training data including sample data each including items for identifying presence or absence of affection of a plurality of diseases and to which label information is attached, the label information indicating whether individuals are affected with any of the diseases, the machine learners respectively including different types of learned models that have learned in advance to determine affection of the same disease, the machine learners configured to output a prediction result as to whether sample data to be determined for disease affection has affected a disease; and
acquiring a final determination result on the basis of a stacking machine learner that has learned in advance to output a final determination result, using the prediction results from the plurality of machine learners as inputs, and configured to output a determination result as to whether the sample data to be determined for affection is affected with a disease on the basis of the prediction results from the plurality of machine learners.

16. A disease affection determination program for causing a computer to realize the processes of:
acquiring a plurality of sample data respectively acquired from individual organisms and including respective expression levels of a plurality of types of biomarkers including miRNA in individual organism-derived samples;
generating a learned model in which presence or absence of affection of a plurality of diseases is determinable, the plurality of diseases being output as a result of machine learning using, as training data, sample data with label information in which items for identifying whether each individual organism has affected the plurality of diseases are provided as label information, for each of the plurality of sample data; and
determining presence or absence of affection of each of the plurality of diseases, using the learned model, for sample data newly acquired from another organism for which affection determination is to be performed.

17. A disease affection determination program for causing a computer to realize the processes of:
acquiring a plurality of sample data respectively acquired from individual organisms and including respective expression levels of a plurality of types of biomarkers including miRNA in an individual organism-derived sample;
generating a learned model in which presence or absence of affection of a predetermined disease is determinable, the predetermined disease being output as a result of machine learning using, as training data, sample data with label information in which items for identifying whether each individual organism is affected with any one of a predetermined group of diseases determined in advance or whether each individual organism is not affected with any of the predetermined group of diseases determined in advance, as information regarding the disease when affected with the disease or information indicating that the individual organism is not affected when not affected, as label information for each of the plurality of sample data; and
determining whether affected with any one of the predetermined group of diseases or whether not affected with any of the predetermined group of diseases, using the learned model, for sample data newly acquired from another organism for which affection determination is to be performed.
